# EUROPEAN PATENT SPECIFICATION

(11) **EP 0 542 424 B1**
(45) Date of publication and mention of the grant of the patent: **27.08.1997**
(21) Application number: 92309239.9
(22) Date of filing: 09.10.1992
(51) Int. Cl.: C12N 15/12, C12N 5/10, C12P 21/08, C07K 14/00

(54) **Oxytocin recepto and DNA coding for the same**
Oxytocin-Rezeptor, dafür kodierende DNS
Récepteur de l'oxytocine et sa séquence

(30) Priority: 09.10.1991 JP 262167/91; 27.03.1992 JP 71258/92
(43) Date of publication of application: 19.05.1993
(60) Divisional application: 97102130.8
(73) Proprietor: ROHTO PHARMACEUTICAL CO., LTD., Ikuno-ku, Osaka 544 (JP)
(72) Inventor: Tanizawa, Osamu, Osaka-shi, Osaka (JP); Saji, Fumitaka, Nishinomiya-shi, Hyogo (JP); Okayama, Hiroto, Mino-shi, Osaka (JP); Azuma, Chihiro, Osaka-shi, Osaka (JP); Kimura, Tadashi, Osaka-shi, Osaka (JP)
(74) Representative: Ritter, Stephen David

(56) References cited:
- 3rd International Vasopressin Conference, Montpellier, FRANCE, August 5-10, 1990.
- NATURE. vol. 356, 9 April 1992, LONDON GB pages 526 - 529 Kimura T;Tanizawa O;Mori K;Brownstein MJ;Okayama H 'Structure and expression of a human oxytocin receptor'.
- ENDOCRINOLOGY vol. 120, no. 6, June 1987, USA pages 2474 - 2482 Soloff MS;Fernstrom MA 'Solubilization and properties of oxytocin receptors from rat mammary gland'.
- ENDOCRINOLOGY vol. 122, no. 5, May 1988, USA pages 1769 - 1772 Soloff MS;Beauregard G;Potier M; 'Determination of the functional size of oxytocin receptors in plasma membranes from mammary gland and uterine myometrium of the rat by radiation inactivation.'

## Description

The present invention relates to a receptor of oxytocin being a posterior pituitary hormone which is widely used as an uterotonic agent in the general clinic field, a DNA sequence coding for the same, a recombinant DNA containing the DNA and a transformant having the recombinant DNA.

Oxytocin being a posterior pituitary hormone has an activity causing uterine contraction and is widely used for induction and enhancement of labor pains in the obstetric clinical field. However, there is a great individual variation in such activity. Also, oxytocin is effective against only the pregnant uterus in the term to show such activity. It has been considered that increase of the oxytocin receptor is essential for myometrium to obtain sensitivity to oxytocin.

Therefore, it is necessary to obtain an oxytocin receptor and a DNA sequence coding for the same, in order to minutely elucidate a series of biological response mechanism between oxytocin and the oxytocin receptor and an expression mode of oxytocin receptor on living tissue, further in order to enable to measure an expression level of the oxytocin receptor before labor or at the time of labor, construct screening method for evaluating oxytocin activity and relieve of labor pains by an antibody against the oxytocin receptor, and in order to obtain the oxytocin receptor, a DNA sequence coding for the same, a recombinant DNA containing the DNA and a transformant having the recombinant DNA. However, studies of isolation, purification and structural analysis of the oxytocin receptor are hardly carried out.

In consideration of such circumstance, the object of the present invention is to obtain an oxytocin receptor, a DNA sequence coding for the same, recombinant DNA containing the DNA and a transformant having the recombinant DNA.

M.S. Soloff et al, in Endocrinology, vol. 122, pages 1769-1772, 1998 describes rat oxytocin receptors purified from rat uterine myometrium and determination of the size of these receptors. Oxytocin receptors from rat mammary glands is described by M.S. Soloff in Collogue Inserm, vol. 208, pages 65-33, 1991.

According to the present invention there is provided an isolated oxytocin receptor comprising the sequence of 389 amino acids presented in SEQ ID NO:1, or a variant thereof, or a portion of the sequence or of the variant.

The variant amino sequence differs from said amino acid sequence of SEQ ID NO: 1 by up to 4 amino acid substitutions, additions or deletions, and in a particular embodiment zero or one substitution, addition or deletion.

In an embodiment of the invention the substitutions to the amino acid sequence of SEQ ID NO: 1 are made according to the following table:-

| ORIGINAL AMINO ACID | SUBSTITUTE |
|---|---|
| Ser | Cys |
| Arg | His or Lys |
| Leu | Ile, Met or Phe |
| Pro | Ala |
| Ala | Pro |
| Val | Met or Ile |
| Ile | Met, Leu, Phe or Val |
| Phe | Met, Tyr, Ile or Leu |
| Tyr | Phe |
| His | Gln or Arg |
| Gln | Glu or His |
| Asn | Asp |
| Lys | Arg |
| Asp | Asn |
| Glu | Gln |
| Met | Ile, Leu, Phe or Val |

The portion comprises at least 350 amino acids of the amino acid sequence of SEQ ID NO: 1 or of the variant , and in a particular embodiment comprises a contiguous sequence of at least 350 amino acid residues contained within the sequence of SEQ ID NO: 1.

According to a second aspect of the invention there is provided a DNA segment coding for the oxytocin receptor of the first aspect of the invention. In an embodiment of the second aspect of the invention, the DNA segment encodes the amino acid sequence given in SEQ ID NO: 1. In a further embodiment of the second aspect of the invention, the segment has the nucleotide sequence represented in SEQ ID NO: 2.

In a still further embodiment of the second aspect there is provided a DNA segment which is at least 75% homologous with the sequence of SEQ ID NO: 2, and in a particular embodiment is substantially homologous with the sequence of SEQ ID NO: 2.

According to a third aspect of the invention there is provided a recombinant DNA molecule comprising a vector containing a DNA according to any of the embodiments of the second aspect of the invention.

According to a fourth aspect of the invention there is provided a DNA that hybridizes to a DNA according to any of the embodiments of the second aspect of the invention.

According to a fifth aspect of the invention there is provided a cell transformed with a DNA according to the third aspect of the invention.

According to a sixth aspect of the invention there is provided a method of producing an oxytocin receptor comprising culturing a cell according to the fifth aspect of the invention under conditions such that said DNA segment is expressed so that said oxytocin receptor is produced, and isolating said oxytocin receptor.

According to a seventh aspect of the invention there is provided a kit comprising an oxytocin receptor or antibody therefor disposed within a container, and a buffer.

As the result of the continuous effort to obtain an oxytocin receptor of the present inventors, it has been found that as a membrane current can be high-sensitively detected the phenomenon wherein a protein encoded by a specific mRNA increases intracellular calcium ion in a perfusion with oxytocin and causes influx of chlorine ion on the oocytes of African clowed toad, Xenopus laevis. Usually a mRNA fractioned by means of sucrose density gradient method for the like is used. However, the starting material for the mRNA is a human pathologic specimen in an extremely specific acosmia, and an amount of the material is finite. It is impossible to take the material again. Therefore, the present inventors have succeeded in cloning a gene coding for a biologically active protein causing membrane current in perfusion with oxytocin on cell membrane, i.e. oxytocin receptor, for the first time by means of preparing cDNA library from whole mRNA by using a plasmid vector and then fractioning the library by size. Consequently the present invention is accomplished.

Hereinafter, the oxytocin receptor, the DNA sequence, the recombinant DNA and the transformant of the present invention are explained.

The oxytocin receptor of the present invention is a polypeptide or protein comprising the whole or a part of the amino acid sequence shown in SEQ ID NO: 1, or a variant thereof, and a biologically active polypeptide or protein causing increase of concentrations of intracellular calcium ion and inositol triphosphate. More particularly, the oxytocin receptor of the present invention is a polypeptide or protein having an amino acid sequence wherein one or plural amino acid residues in the amino acid sequence shown in SEQ ID NO: 1 are substituted, or one or plural amino acid residues are lacking, further, or one or plural amino acid residues are added to the amino acid sequence shown in SEQ ID NO: 1.

The DNA sequence of the present invention is a DNA sequence coding for the oxytocin receptor of the present invention. Examples of the DNA sequence of the present invention are, for instance, a DNA sequence comprising the whole or a part of the base sequence shown in SEQ ID NO: 2, or a varied DNA thereof. More particularly, there can be exemplified a DNA sequence which has a DNA sequence wherein one or plural nucleotides in DNA sequence shown in SEQ ID NO: 2 are substituted with other nucleotides, or one or plural nucleotides are lacking, further, or one or plural nucleotides are added to the DNA sequence shown in SEQ ID NO: 2.

The recombinant DNA of the present invention is a DNA containing the DNA sequence of the present invention in a suitable expression vector. The recombinant DNA of the present invention may contain a sequence for regulating an expression which is functionally combined with the DNA sequence of the present invention. The vector used in the present invention is not particularly limited, when the vector can be replicated in a suitable host and can be expressed. As examples of the vector used in the present invention, pcD vector, the varied pcD vector explained in Example 1 described below, can be exemplified.

The present invention is more specifically described and explained by means of the following Example. It is to be understood that the present invention is not limited to the Example.

### Example 1

Human myometrium tissue which could be accidentally obtained (pathologic specimen of the case wherein extensive bleeding was encountered by uterine rupture just after the vaginal delivery and the whole uterus was obliged to removed) was rapidly freezed in liquid nitrogen and stored at -70°C.

### (1) Size estimation of mRNA coding for the oxytocin receptor

The human myometrium tissue was powdered in liquid nitrogen. The powdered tissue was homogenized in a solution containing 5.5 M guanidium isothiocyanate, 100 mM 2-mercaptoethanol, 0.5 % sodium N-laurylsarcosine and 2.5 mM sodium citrate (pH 7.0), layered onto the cushion of the solution containing 5.7 M CsCℓ and 0.1 M EDTA (pH 7.0) and ultracentrifuged to recover whole RNA as precipitation. Thus obtained precipitation of whole RNA was purified by repeating precipitation with ethanol. From the whole RNA was isolated polyadenyrated RNA by using oligo-dT cellulose column.

In the solution containing 10 mM Tris-HCℓ and 1 mM EDTA (pH 7.0) was dissolved 160 µg of the polyadenyrated RNA. After performing heat-denaturation, the solution with 0.1 % sodium N-laurylsarcosine was layered onto the discontinuous sucrose density-gradient (8 layers, 5 %-25 % sucrose) and ultracentrifuged at 35,000 rpm for 7.5 hours. The ultracentrifuged layers were fractionated from low density of sucrose by means of a fraction collecter to give 25 fractions of 0.4 mℓ. Each size fractionated mRNA was recovered by precipitation with ethanol. The whole mRNA was dissolved in water in a ratio of 1 mg/mℓ, and the size fractionated mRNA was dissolved in 5 µℓ of water, to give samples for microinjection into oocytes of Xenopus. The samples were injected into the Xenopus oocytes treated at room temperature for 3 hours with the modified Barth's medium from which calcium ion was removed (refer to Transcription and Translation Edt. B. Hames, S. Higgins IRL Press. 1984. Oxford, pp 271-301) wherein 2 mg/mℓ collagenase was dissolved. Into the Xenopus oocytes was injected 50 nℓ of each size fractionated mRNA by means of a hydraulic microsyringe equipped with a microcapillary. These oocytes were cultured in the modified Barth's medium at 19°C for 72 hours.

Then, according to the method of Dascal et al. (refer to J. Physiol 366, 299-313, (1985)), into these oocytes were inserted 2 microelectrodes filled with 3M KCℓ. One electrode was electrified to settle a membrane potential to -60 mV. The other was attached to a system for measuring a membrane current. This oocyte had been perfused with Normal Ringer's Solution (115 mM NaCℓ, 1 mM KCℓ, 1.8 M CaCℓ₂ and 5 mM Tris pH 7.4) and then perfused with 1 µM oxytocin for only 1 minute. In case of 1 µM oxytocin, a membrane current was observed in the both oocyte into which the whole mRNA was injected and the oocyte into which the 19th to the 21st fractions of the size fractionated mRNA was injected. It is inferred that an oxytocin receptor capable of binding to oxytocin was expressed on the oocytes because a membrane current was caused by the perfusion with oxytocin. That is, such fact indicates that a mRNA of about 4-5 kb (killbases) capable of showing activity of the oxytocin receptor exists in human myometrium tissue in term.

### (2) Preparation of cDNA library

According to the method of Okayama et al. (refer to Methods in Enzymology 154, 3-28, (1987)), the whole RNA of human myometrium was purified. From the purified RNA was obtained the whole mRNA by means of an oligo(dT)-cellulose column.

As a vector for preparing a library was used a varied vector produced by M. Brownstein of NIH, U.S.A. (unpublished). The varied vector is a vector obtained by improving the expression vector pcD for animal cells developed by Okayama-Berg (refer to Hiroto Okayama and Paul Berg "Molecular cell Biology" 3, 280 (1983) and 2, 161 (1982)). The varied vector is the vector primer pcDV1 modified by inserting T7 RNA polymerase promoter and sites to be cleaved by restriction enzymes NotI, BstXI and XbaI therein. The linker used in the present invention is the linker pL1 wherein promoter region of SP6 RNA polymerase was inserted.

This vector primer and linker were provided by Mr. Brownstein of NIH, U.S.A. in a condition that to the vector primer and the linker were added dG tail and dT tail, respectively by means of terminal deoxynucleotidyl transferase (TdT) and then purified by means of a low melting agarose and oligo(dA)-cellulose column.

By using 7 µ g of the purified whole mRNA was synthesized cDNA of the first strand according to the above-mentioned Methods in Enzymology, 154, 3-28, (1987). The cDNAs of the average length 630 bp were synthesized by this reaction. Then, this cDNA was tailed with dC by reacting with 10 units of TdT at 37°C for 5 minutes in the presence of dCTP. Thus obtained mRNA-cDNA hybrid vector primers were digested and reacted with 28 units HindIII at 37°C for 1 hour.

Successively, the 1/10 amount of this mRNA-cDNA hybrid vector primers digested with HindIII were mixed and annealed with 0.25 pM the varied pL1 linker which was previously prepared. After annealing, the hybrid vector primers with the linker were looped by reacting with 100 units of DNA ligase (derived from E. coli) in the presence of 0.1 mM β-NAD at 12°C overnight. The looped mRNA-cDNA hybrid was reacted in the presence of 40 µM dNTP with 30 units of RNase H, 50 units of DNA ligase (derived from E. coli) and 3 units of DNA polymerase I at 12°C for 60 minutes, and successively at room temperature for 60 minutes to be converted into a double-stranded cDNA. Thereby cDNA library derived from human myometrium was constructed in the varied pcD vector.

Subsequently cDNA library was transformed into a high efficience competent cells of E. coli DH5 which was prepared according to the method developed by Inoue and Okayama (refer to Gene 96, 23-28 (1990)) to give 4 x 10⁷ cells of independent transformants. After proliferating this transformants in L-Broth medium containing 100 µg/mℓ ampicillin, plasmids were isolated to obtain a plasmid library according to a standard method for preparing plasmid DNA.

### (3) Preparation of sublibrary

As described in (1), cDNA coding for an oxytocin receptor is about 4 to 5 kbp or so. The varied pcD vector is about 3 kbp. Therefore, with 80 units of each of NotI and PvuI was digested 40 µg of each this cDNA library, respectively, and thus obtained linear cDNA was separated into each linear cDNA having a length of from 7 kbp to 23 kbp by electrophoresis using low melting agarose gel. Each of thus obtained separated linear cDNA was reacted with 1.2 units of T4 DNA ligase in the presence of 66 µM ATP in a reaction system of 200 µℓ at 4°C overnight to be looped again. The sublibrary (NotI sublibrary), which was looped again after digesting with NotI, contained 60,000 to 90,000 clones. The sublibrary (PvuI sublibrary), which was looped again after digesting with PvuI, contained about 600,000 clones.

### (4) Transcription in vitro

### (a) Preparation of template DNA

The 1/20 amount of the NotI sublibrary and the 1/200 amount of the PvuI sublibrary, which were obtained in (3), were introduced into DH5 respectively to give transformants. As to these transformants, 8 pools respectively, 16 pools in total were proliferated in 50 mℓ of L-Broth medium containing 100 µg/mℓ ampicillin until the OD⁶⁰⁰ becomes about 1 (2 mℓ of each culture solution was taken and freezed for preservation in the presence of 7 % DMSO). Thereafter 7.5 mg of chloramphenicol was added to the culture solution and further culturing was performed for 16 hours. The cells were collected, and plasmids were isolated according to a standard method for preparing plasmid DNA. Ten µg of each pool prepared from the NotI sublibrary (N1-N8) were digested with 12 units of NotI at 37°C for 60 minutes to give linear DNAs. Also, 10 µg of each pool prepared from the PvuI sublibrary (P1-P8) were digested with 12 units of BstXI at 50°C for 90 minutes to give linear DNAs. These were purified by a conventional treatment with phenol/chloroform and precipitation with ethanol. The purified cDNAs were dissolved in a solution containing 10 Mm Tris (pH 7.0) and 1 mM EDTA so as to give a concentration of 1µg/µℓ. Thus obtained solution was used as a solution of template DNA.

### (b) Transcription in vitro

Five units of SP6 RNA polymerase (Bethesda Research Laboratories) was added to 25 µℓ of the reaction liquid consisting of the constitution described below (40 mM Tris (pH 7.9), 6 mM MgCℓ₂ and 2 mM spermidine hydrochloride as a buffer, and a substrate (a mixture of ATP, CTP, GTP and UTP), m7G (5') ppp (5')G and DTT of a mRNA caping kit of Stratagen Cloning Systems were used). Then, using 5 µg of each template DNA obtained in (a), the mixture was reacted at 39°C for 90 minutes. Further, thereto was added 10 units of RNase-free DNaseI (Stratagen Cloning Systems) and the reaction was carried out at 37°C for 5 minutes to decompose the template DNA. Successively, the reaction liquid was applied to Sephadex G-50 spun column (Boehringer Mannheim GmbH). The reaction liquid was passed through the column by centrifuging at 2200rpm for 5 minutes and an excess substrate was removed. Further this reaction liquid was purified by a conventional treatment with phenol/chloroform and precipitation with ethanol. About 2-3 µg of each synthesized mRNA was recovered.

### (5) Measurement of oxytocin receptor activity

In 2.5 µℓ of water was dissolved each of 16 pools of the synthesized mRNA obtained in (4)-(b). By means of the technique described in (1), thus obtained solution was injected into 15-20 oocytes of Xenopus per pool. The oocytes were cultured at 19°C for 3 days in the modified Barth's medium. Then, according to the method described in (1) these oocytes into which the mRNA derived from each pool were injected were perfused, and change of a membrane current was examined in case of perfusing with 1 µM oxytocin for 1 minute. In the oocytes into which one pool derived from the NotI sublibrary among the pools prepared in (4) was injected, was detected a membrane current caused by oxytocin. On the ground that a membrane current was caused by perfusion with oxytocin, it is inferred that in an oxytocin receptor capable of binding to oxytocin is expressed in the oocyte. Because the pool consisted of about 6,000 clones, a part of the cells being obtained and stored in (4)-(a) were diluted, and 8 pools containing 1,500 clones were prepared. According to the method described in (4), a template DNA was prepared, and a synthesized mRNA was prepared by transcription in vitro. Subsequently, screening wherein injection into Xenopus oocytes was performed, was repeated again. As a result, 4 positive pools as to an oxytocin receptor activity were obtained. These positive pools were selected by the above-mentioned screening to finally give 2 clones showing an oxytocin receptor activity. As the result of digestion with restriction enzymes, these two clones were found to be identical. The length of cDNA thereof was about 4 kbp.

This cDNA was subjected to sequencing according to dideoxy method of Sanger et al. Amino acid sequence deduced from the determined base sequence (this cDNA is in a form of double strand) is shown in SEQ ID NO: 1.

### (6) Study of specificity of expressed oxytocin receptor

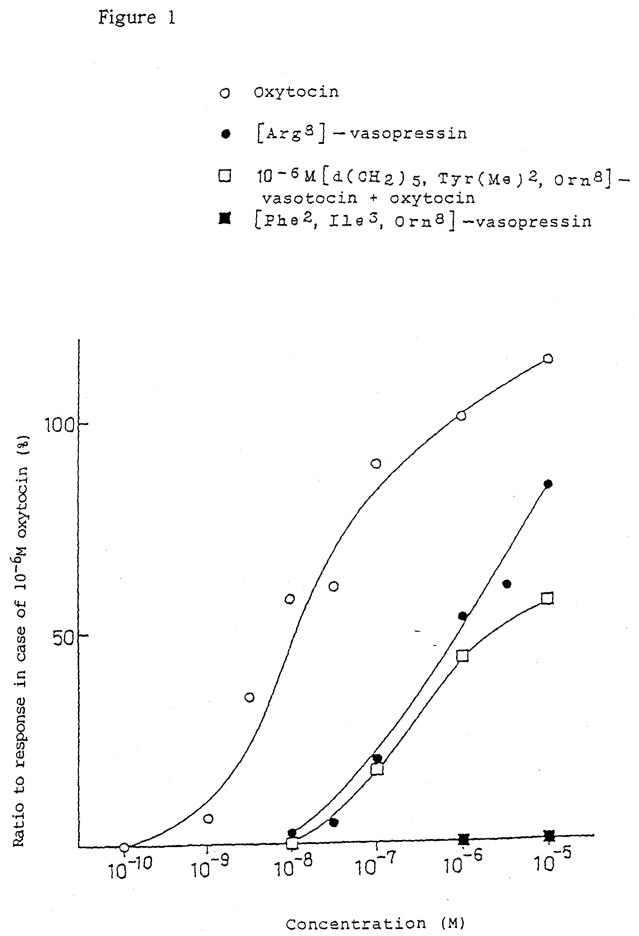

The single clone of cDNA of an oxytocin receptor obtained in (5) was again amplified and purified in a form of plasmid. Then, 10 µg of the plasmid was digested with ristriction enzyme NotI to give a linear DNA. A synthesized mRNA was obtained using 1 µg of the linear DNA as a template according to the method described in (4). The mRNA was injected into Xenopus oocyte in amount of 2-5 ng per oocyte, and the injected oocytes were cultured. These oocytes were perfused with each of 10⁻¹⁰M-10⁻⁵M oxytocin and 10⁻⁸M-10⁻⁵M Arg⁸-vasopressin for 1 minute, and dimensions of the membrane current thereof were compared. Each ratio of a current to the detected current during the perfusion with 10⁻⁶M oxytocin regarded as 100 % was shown in Fig. 1. As is clear from Fig. 1, Arg⁸-vasopressin could not cause approximately the same membrane current in case of oxytocin until the concentration in perfusion is nearly hundred times as high as that of oxytocin. Also, as to concentrations of both substances required so as to cause a current equal to 50 % of the largest current, in case of oxytocin, the concentration was about 10⁻⁸M and close to a physiological concentration. In contrust, that of the vasopressin was about 10⁻⁶M Therefore, it was found that the cDNA isolated by us is a cDNA coding for a receptor specific to oxytocin. Also, in case of previously perfusing for 1 minute with 10⁻⁶M vasotocin known to be a specific antagonist to oxytocin ([d(CH₂)₅, Tyr(Me)², Orn⁸]-vasotocin) (refer to K. Bankowski et al., Int. J. Peptide Protein Res. 16, 382 (1980)) just before perfusion with oxytocin, a membrane current caused immediately thereafter by perfusion with oxytocin decreased to not more than 50 % of the largest current in case of 10⁻⁶M oxytocin. Further, using 10⁻⁶M or 10⁻⁵M of [Phe², IIe³, Orn⁸]-vasopressin being a vasopressin selective agonist, no response was observed. Such observations indicate that cDNA obtained by us codes for a receptor capable of specifically reacting with oxytocin and binding to oxytocin.

Because there were obtained the receptor, the DNA sequence, the recombinant DNA and the transformant of the present invention:
1) It comes to be possible to infer labor time from expression level of an oxytocin receptor by using uterine endometrium cells by reason that oxytocin receptors increase on uterine endometrium cells just before delivery.
2) It comes to be possible to scientifically and semi-quantitativelly predict whether or not relief of labor pains of patients with preterm labor is possible by reason that oxytocin receptors increase even in the preterm labor cases.
3) Prevention of hypertonic uterine dysfunction or determination of time of inducing labor pains can be carried out on the objective grounds by previously estimating oxytocin receptor level before using an agent inducing labor pains.
4) Expression of the receptor of the present invention in an animal cells or a Xenopus oocyte enables screening for estimation of an oxytocin activity.
5) Lelief of labor pains comes to be possible by preparing an antibody against the receptor of the present invention and inhibiting binding to oxytocin thereby.

### BRIEF DESCRIPTION OF THE DRAWINGS

### Figure 1

A graph showing concentration of substances perfused on a cross axis (concentration of oxytocin in case of 10⁻⁶M vasotocin + oxytocin) and each ratio of a current to the detected current during the perfusion with 10⁻⁶M oxytocin regarded as 100 % on a vertical axis.

The clones referred to at (5) above, that is, JM 103 derived from E.coli K12 into which recombinant DNA was introduced, have been deposited with the Fermentation Research Institute (Deposit No. Bikouken 12907 (FERM P-12907)).

### SEQUENCE ID NO: 1

- SEQUENCE LENGTH :: 389 amino acids
- SEQUENCE TYPE :: Amino acid
- MOLECULE TYPE :: Protein
- HYPOTHETICAL SEQUENCE :: Yes
- ORIGINAL SOURCE ORGANISM :: Homo Sapiens
- TISSUE TYPE SEQUENCE:: Myometrium tissue


1               5                   10                  15

            20                  25                  30

        35                  40                  45

    50                  55                  60

65                  70                  75                  80

                85                  90                  95

            100                 105                 110

        115                 120                 125


    130                 135                 140

145                 150                 155                 160

                165                 170                 175

            180                 185                 190

        195                 200                 205

    210                 215                 220

225                 230                 235                 240

                245                 250                 255

            260                 265                 270

        275                 280                 285

    290                 295                 300

305                 310                 315                 320

                325                 330                 335

            340                 345                 350


        355                 360                 365

    370                 375                 380

385

### SEQ ID NO: 2

- SEQUENCE LENGTH :: 1167 nucleotides
- SEQUENCE TYPE :: nucleic acid
- STRANDENESS :: double stranded
- MOLECULE TYPE :: cDNA to mRNA
- HYPOTHETICAL SEQUENCE :: No
- ORIGINAL SOURCE ORGANISM :: Homo Sapiens
- TISSUE TYPE SEQUENCE :: Myometrium tissue

ATG GAG GGC GCG CTC GCA GCC AAC TGG AGC GCC GAG GCA GCC AAC GCC 48

1               5                   10                  15
AGC GCC GCG CCG CCG GGG GCC GAG GGC AAC CGC ACC GCC GGA CCC CCG 96

            20                  25                  30
CGG CGC AAC GAG GCC CTG GCG CGC GTG GAG GTG GCG GTG CTG TGT CTC 144

        35                  40                  45
ATC CTG CTC CTG GCG CTG AGC GGG AAC GCG TGT GTG CTG CTG GCG CTG 192

    50                  55                  60
CGC ACC ACA CGC CAG AAG CAC TCG CGC CTC TTC TTC TTC ATG AAG CAC 240

65                  70                  75                  80

CTA AGC ATC GCC GAC CTG GTG GTG GCA GTG TTT CAG GTG CTG CCG CAG 288

                85                  90                  95
TTG CTG TGG GAC ATC ACC TTC CGC TTC TAC GGG CCC GAC CTG CTG TGC 336

            100                 105                 110
CGC CTG GTC AAG TAC TTG CAG GTG GTG GGC ATG TTC GCC TCC ACC TAC 384

        115                 120                 125
CTG CTG CTG CTC ATG TCC CTG GAC CGC TGC CTG GCC ATC TGC CAG CCG 432

    130                 135                 140
CTG CGC TCG CTG CGC CGC CGC ACC GAC CGC CTG GCA GTG CTC GCC ACG 480

145                 150                 155                 160
TGG CTC GGC TGC CTG GTG GCC AGC GCG CCG CAG GTG CAC ATC TTC TCT 528

                165                 170                 175
CTG CGC GAG GTG GCT GAC GGC GTC TTC GAC TGC TGG GCC GTC TTC ATC 576

            180                 185                 190
CAG CCC TGG GGA CCC AAG GCC TAC ATC ACA TGG ATC ACG CTA GCT GTC 624

        195                 200                 205
TAC ATC GTG CCG GTC ATC GTG CTC GCT ACC TGC TAC GGC CTT ATC AGC 672

    210                 215                 220
TTC AAG ATC TGG CAG AAC TTG CGG CTC AAG ACC GCT GCA GCG GCG GCG 720

225                 230                 235                 240

GCC GAG GCG CCA GAG GGC GCG GCG GCT GGC GAT GGG GGG CGC GTG GCC 768

                245                 250                 255
CTG GCG CGT GTC AGC AGC GTC AAG CTC ATC TCC AAG GCC AAG ATC CGC 816

            260                 265                 270
ACG GTC AAG ATG ACT TTC ATC ATC GTG CTG GCC TTC ATC GTG TGC TGG 864

        275                 280                 285
ACG CCT TTC TTC TTC GTG CAG ATG TGG AGC GTC TGG GAT GCC AAC GCG 912

    290                 295                 300
CCC AAG GAA GCC TCG GCC TTC ATC ATC GTC ATG CTC CTG GCC AGC CTC 960

305                 310                 315                 320
AAC AGC TGC TGC AAC CCC TGG ATC TAC ATG CTG TTC ACG GGC CAC CTC 1008

                325                 330                 335
TTC CAC GAA CTC GTG CAG CGC TTC CTG TGC TGC TCC GCC AGC TAC CTG 1056

            340                 345                 350
AAG GGC AGA CGC CTG GGA GAG ACG AGT GCC AGC AAA AAG AGC AAC TCG 1104

        355                 360                 365

TCC TCC TTT GTC CTG AGC CAT CGC AGC TCC AGC CAG AGG AGC TGC TCC 1152

    370                 375                 380
CAG CCA TCC ACG GCG 1167

385

## Claims

1. An isolated oxytocin receptor comprising the sequence of 389 amino acids represented in SEQ ID NO:1.

2. An isolated oxytocin receptor comprising an amino acid sequence that is a variant of the sequence of 389 amino acids of SEQ ID NO: 1 in that at least 1 but no more than 4 amino acids are substituted, added or deleted.

3. An oxytocin receptor according to Claim 2 wherein said variant amino acid sequence differs from said amino acid sequence of SEQ ID NO:1 in that only 1 amino acid is substituted, added or deleted.

4. An oxytocin receptor according to Claim 2 or 3 in which the amino acids of SEQ ID NO: 1, if substituted, are substituted according to the following table:
| ORIGINAL AMINO ACID | SUBSTITUTE |
|---|---|
| Ser | Cys |
| Arg | His or Lys |
| Leu | Ile, Met or Phe |
| Pro | Ala |
| Ala | Pro |
| Val | Met or Ile |
| Ile | Met, Leu, Phe or Val |
| Phe | Met, Tyr, Ile or Leu |
| Tyr | Phe |
| His | Gln or Arg |
| Gln | Glu or His |
| Asn | Asp |
| Lys | Arg |
| Asp | Asn |
| Glu | Gln |
| Met | Ile, Leu, Phe or Val |

5. An isolated oxytocin receptor comprising a 350 amino acid portion of an oxytocin receptor according to any preceding claim.

6. An oxytocin receptor according to Claim 1 consisting of SEQ ID NO:1.

7. An isolated DNA encoding an oxytocin receptor according to any of Claims 1 to 6.

8. A DNA according to Claim 7 comprising SEQ ID NO:2.

9. A DNA according to Claim 7 which is at least 75% homologous with the sequence of SEQ ID NO:2.

10. A recombinant DNA comprising a vector containing a DNA according to any of Claims 7 - 9.

11. A DNA that hybridizes to a DNA according to any of Claims 7 to 9.

12. A cell transformed with a DNA according to Claim 10.

13. A method of producing an oxytocin receptor comprising culturing a cell according to Claim 12 under conditions such that said DNA is expressed so that said oxytocin receptor is produced, and isolating said oxytocin receptor.

14. A kit comprising, disposed in a container, an oxytocin receptor or an antibody therefor and a buffer.

## Patentansprüche

1. Isolierter Oxytocinrezeptor, umfassend die in SEQ ID Nr. 1 dargestellte Sequenz von 389 Aminosäuren.

2. Isolierter Oxytocinrezeptor, umfassend eine dahingehende Variante der in SEQ ID Nr.1 dargestellten Sequenz von 389 Aminosäuren, daß mindestens eine, aber nicht mehr als vier Aminosäuren substituiert, zugefügt oder deletiert sind.

3. Oxytocinrezeptor nach Anspruch 2, bei dem sich die variierte Aminosäuresequenz von der Aminosäuresequenz nach SEQ ID Nr.1 dadurch unterscheidet, daß nur eine Aminosäure substituiert, zugefügt oder deletiert wurde.

4. Oxytocinrezeptor gemäß Anspruch 2 oder 3, bei dem die Aminosäuren aus SEQ ID Nr.1, wenn sie substituiert werden, gemäß der folgenden Tabelle ersetzt werden:
| ursprüngliche Aminosäure | Ersatz |
|---|---|
| Ser | Cys |
| Arg | His or Lys |
| Leu | Ile, Met or Phe |
| Pro | Ala |
| Ala | Pro |
| Val | Met or Ile |
| Ile | Met, Leu, Phe or Val |
| Phe | Met, Tyr, Ile or Leu |
| Tyr | Phe |
| His | Gln or Arg |
| Gln | Glu or His |
| Asn | Asp |
| Lys | Arg |
| Asp | Asn |
| Glu | Gln |
| Met | Ile, Leu, Phe or Val |

5. Isolierter Oxytocinrezeptor, der einen Teil mit 350 Aminosäuren des Oxytocinrezeptors gemäß einem der vorstehenden Ansprüche umfaßt.

6. Oxytocinrezeptor gemäß Anspruch 1, bestehend aus SEQ ID Nr.1

7. Isolierte DNS, die für einen Oxytocinrezeptor gemäß einem der Ansprüche 1 bis 6 codiert.

8. DNS gemäß Anspruch 7, die SEQ ID Nr.2 umfaßt.

9. DNS gemäß Anspruch 7, die zu mindestens 75 % der Sequenz von SEQ ID Nr.2 homolog ist.

10. Rekombinante DNS, enthaltend einen Vektor, der eine DNS gemäß einem der Ansprüche 7 bis 9 enthält.

11. DNS, die mit der DNS gemäß einem der Ansprüche 7 bis 9 hybridisiert.

12. Mit der DNS gemäß Anspruch 10 transformierte Zelle.

13. Verfahren zur Herstellung eines Oxytocinrezeptors, bei dem man eine Zelle gemäß Anspruch 12 und solchen Bedingungen kultiviert, daß die DNS exprimiert wird, so daß der Oxytocinrezeptor hergestellt wird, und den Oxytocinrezeptor isoliert.

14. Kit, der, in einem Behälter angeordnet, einen Oxytocinrezeptor oder einen Antikörper dagegen und einen Puffer enthält.

## Revendications

1. Récepteur d'oxytocine isolé comprenant la séquence de 389 acides aminés représentée dans SEQ ID NO :1.

2. Récepteur d'oxytocine isolé comprenant une séquence d'acides aminés qui est une variante d'une séquence de 389 acides aminés de SEQ ID NO:1 dans laquelle au moins 1, mais pas plus de 4 acides aminés sont substitués, additionnés, ou délétés.

3. Récepteur d'oxytocine selon la revendication 2, dans lequel ladite variante d'acides aminés diffère de la séquence d'acides aminés de SEQ ID NO :1 en ce qu'un seul acide aminé est substitué, additionné, ou délété.

4. Récepteur d'oxytocine selon la revendication 2 ou la revendication 3, dans lequel les acides aminés de SEQ ID NO :1, s'ils sont substitués, le sont selon le tableau suivant :
| ACIDE AMINE ORIGINAL | SUBSTITUT |
|---|---|
| Ser | Cys |
| Arg | His, ou Lys |
| Leu | Ile, Met, ou Phe |
| Pro | Ala |
| Ala | Pro |
| Val | Met ou Ile |
| Ile | Met, Leu, Phe, ou Val |
| Phe | Met, Tyr, Ile, ou Leu |
| Tyr | Phe |
| His | Gln, ou Arg |
| Gln | Glu, ou His |
| Asn | Asp |
| Lys | Arg |
| Asp | Asn |
| Glu | Gln |
| Met | Ile, Leu, Phe, ou Val |

5. Récepteur d'oxytocine isolé comprenant une portion de 350 acides aminés, d'un récepteur d'oxytocine selon l'une quelconque des revendications précédentes.

6. Récepteur d'oxytocine selon la revendication 1, consistant en la séquence de SEQ ID NO :1.

7. ADN isolé encodant un récepteur d'oxytocine selon l'une quelconque des revendications 1 à 6.

8. ADN selon la revendication 7, comprenant la séquence de SEQ ID NO :2.

9. ADN selon la revendication 7, qui est au moins à 75 % homologue de la séquence de SEQ IN NO :2.

10. ADN recombinant comprenant un vecteur contenant un ADN selon l'une quelconque des revendications 7 à 9.

11. ADN qui hybride en un ADN selon l'une quelconque des revendications 7 à 9.

12. Cellule transformée avec un ADN selon la revendication 10.

13. Procédé de production d'un récepteur d'oxytocine comprenant la culture d'une cellule selon la revendication 12, à condition que ledit ADN soit exprimé de façon à ce que ledit récepteur d'oxytocine soit produit, et l'isolement dudit récepteur d'oxytocine.

14. Kit comprenant, disposés dans un container, un récepteur d'oxytocine, ou un anticorps à cet effet, et un tampon.
